# EUROPÄISCHE PATENTANMELDUNG

(11) **EP 1 271 142 A1**
(43) Veröffentlichungstag der Anmeldung: **02.01.2003**
(21) Anmeldenummer: 02010988.0
(22) Anmeldetag: 17.05.2002
(51) Int. Cl.: G01N 33/34, G01B 21/30, G01B 5/28, G01B 13/22

(54) **Vorrichtung und Verfahren zur Bestimmung der Glätte von Papier**

(30) Priorität: 25.06.2001 DE 10130009
(71) Anmelder: ROSCHIWAL + PARTNER ELECTRONIC SYSTEME GMBH, 86179 Augsburg (DE)
(72) Erfinder: Popp, Konrad Joseph, 86199 Augsburg (DE); Refle, Franz, 86637 Villenbach (DE)
(74) Vertreter: Rapp, Bertram, Dr.

(57) **Zusammenfassung**

Beschrieben wird eine Vorrichtung und ein automatisiertes Verfahren zur Bestimmung der Glätte der Oberfläche einer Papierprobe. Die Vorrichtung enthält eine ebene, die Probe (P) tragende Probenaufnahmefläche (4), innerhalb welcher sich eine zu einem evakuierbaren Behälter (2) führende Bohrung (11) befindet, und einen auf die gegenüberliegende Oberfläche der Papierprobe (P) wirkenden, abdichtend ausgebildeten Druckstempel (1) zur Erzeugung eines konstanten Anpressdrucks der Probe (P) auf die Probenaufnahmefläche (4). Das Volumen des Behälters (2) ist veränderbar, so daß mittels Vergrößerung des Volumens ein Unterdruck erzeugt werden kann.

## Beschreibung

Die Erfindung betrifft eine Vorrichtung und ein Verfahren zur Bestimmung der Glätte der Oberfläche einer Papierprobe.

Aus dem Stand der Technik bekannt ist die Bestimmung der Glätte von Papier nach Bekk. In der DIN-Norm 53 107 ist der Zweck und der Anwendungsbereich dieser Messmethode beschrieben.

Sie ist für einen weiten Bereich von Papieren, Kartons und Pappen anwendbar. Die Glätte nach Bekk, hängt von der Form, dem Gesamtvolumen und der Verteilung der Hohlräume ab, die zwischen Papieroberfläche und ideal gedachter Ebene unter den festgelegten Kontaktbedingungen entstehen. Je größer die Bekk-Glätte-Zahl, desto höher ist die Glätte.

Nach der genannten DIN-Norm ist die Glätte nach Bekk die Zeit in Sekunden, die benötigt wird, um eine bestimmte Luftmenge aus der Umgebungsluft bei definierter Druckdifferenz zwischen einer Papieroberfläche und einer ringförmigen, nahezu vollkommenen ebenen Fläche radial nach innen und festgelegten Kontaktbedingungen hindurch zu saugen. Die zu prüfenden Probestücke dürfen hierbei keine Falten, Knicke, Knitterstellen, Löcher, Wasserzeichen, Aufdrucke oder andere Unregelmäßigkeiten aufweisen und auch vor der Messung nicht mit den Fingern berührt werden. Ferner dürfen Sie nicht in unzulässigerweise vorbehandelt, beispielsweise getrocknet werden.

Das bei der Glättemessung nach Bekk benutzte und in der genannten DIN-Norm beschriebene Gerät weist einen Amboß auf, der eine Glasplatte als Messfläche trägt. Diese Glasplatte ist ringförmig, also in ihrem Innenbereich offen, wobei die Öffnung zu einer Bohrung in dem Amboß führt. Diese Bohrung führt wiederum zu einem Vakuumbehälter. Auf die Glasplatte wird die zu prüfende Probe mit der zu prüfenden Oberfläche in Richtung der Glasplatte aufgelegt und mit einem auf die gegenüberliegenden Oberfläche der Probe wirkenden Druckteller angedrückt. Der Druckteller ist mit einer Gummiplatte versehen, um das hindurchtreten von Luft entlang der nicht zu prüfenden Oberseite der Probe zu vermeiden und um einen konstanten Anpressdruck zu erzeugen.

Die Flächenpressung beträgt 100 kPa bei einer Glasplatte mit einer Kontaktfläche von 10 cm². Die zentrische Bohrung in der polierten Glasplatte soll sich wahlweise mit einem von zwei Behältern verbinden und wieder abschalten lassen. Das Volumen der Behälter muß zusammen mit dem der Verbindungsleitung, gerechnet bis zur Oberfläche der Glasplatte, 380 bzw. 38 ml betragen. Die Behälter sind auf einem Überdruck kleiner oder gleich - 533 mbar entlüftbar und lassen sich vakuumdicht verschließen. Ein Druckmessgerät ist vorgesehen, um den Unterdruck innerhalb des Behälters anzuzeigen, insbesondere die bei der Prüfung relevanten Druckwerte - 507, - 480 und - 293 mbar.

Die Volumenverhältnisse sind so gewählt, daß bei einem Anstieg des Überdrucks von - 507 auf - 580 mbar in dem großen Behälter 10 ml und in dem kleinen Behälter 1 ml Außenluft eingedrungen ist. Bei einem Anstieg des Drucks im großen Behälter von - 507 auf - 293 mbar sind 80 ml Außenluft eingedrungen. Die zwischen der Papieroberfläche und der polierten Glasplatte in deren Bohrung eingesaugte Luftmenge von 10 ml oder 80 ml muß also durch die Druckdifferenzen in den Vakuumbehältern bestimmt werden können, wobei die für das Durchsaugen der Luftmenge erforderliche Zeit gemessen wird.

Für die Ausbildung der bevorzugt verwendeten Glasplatte, des Drucktellers und der Gummiplatte sowie des Vakuumbehälters und des Druckmessgeräts wird verwiesen auf die DIN 53 107, Ziffer 6.2, zur Messung des durchgesaugten Luftvolumens auf die Ziffer 6.3.4 dieser Norm und zur Durchführung des Verfahrens auf Ziffer 7 der genannten DIN-Norm.

In der Praxis hat es sich als nachteilig herausgestellt, daß die bekannten Vorrichtungen eine Reihe von Schläuche und Ventilen aufweisen, welche zu Undichtigkeiten des Systems führen, was wiederum zu häufig nicht erkannten Fehlmessungen führt. Ferner sind die Messzeiten relativ lang, und zwar unabhängig von der zu messenden Probe, da bei sehr glatten Papieren häufig auch kürzere Messzeiten zu gleich guten Resultaten führen würden.

Es besteht daher die Aufgabe, eine Vorrichtung und ein Verfahren bereit zu stellen, welches eine schnelle, genaue und zuverlässige Messung der Glätte der Oberfläche von Papier ermöglicht.

Gelöst wird diese Aufgabe mit den kennzeichnenden Merkmalen der Ansprüche 1 bzw. 5 und 6. Vorteilhafte Ausführungsformen sind den jeweiligen Unteransprüchen entnehmbar.

Ein Ausführungsbeispiel der Erfindung wird im folgenden unter Bezugnahme auf die begleitende Zeichnung näher beschrieben, welche einen Querschnitt durch eine erfindungsgemäße Vorrichtung zeigt.

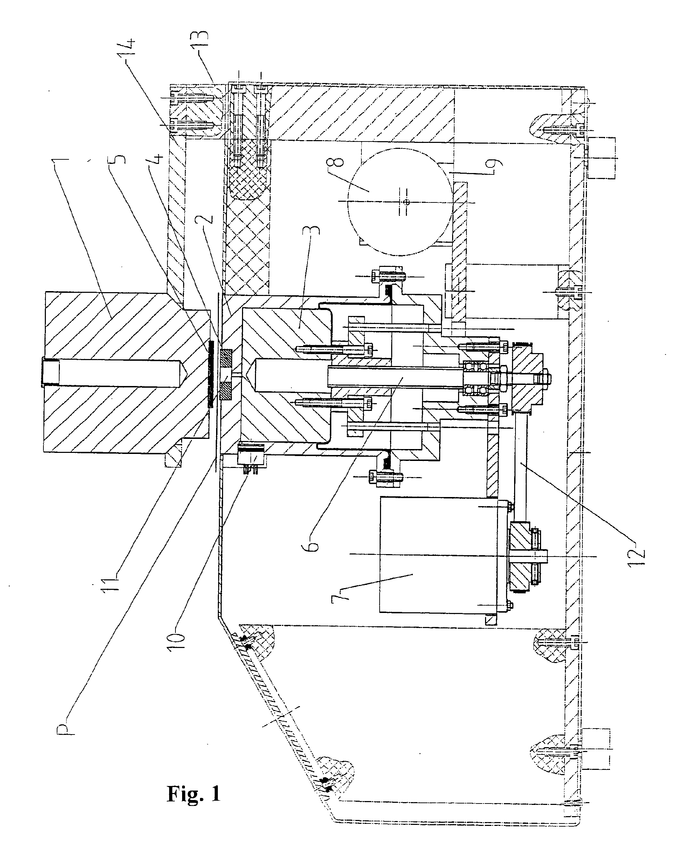

Die in der Figur dargestellte Vorrichtung weist eine Gehäuse 13 auf, an dessen Oberseite über einen Tragarm 14 der Druckstempel 1 fixiert, also relativ zum Gehäuse 13 fest angeordnet ist. Der Druckstempel 1 weist an seiner Unterseite eine Gummiplatte 5 zum Zwecke der Abdichtung auf.

Innerhalb des Gehäuses 13 befindet sich eine Anordnung aus Probenaufnahmefläche 4, Behälter 2, Messkolben 3, Schrittmotor 7 und Antrieb 12. Diese Anordnung weist im wesentlichen die Probenaufnahmefläche 4 auf, welche der Gummiplatte 5 des Druckstempels 1 gegenüberliegt und - bei abgesenktem Druckbehälter 2 - durch einen Zwischenraum von diesem getrennt ist. Dieser Zwischenraum dient der Aufnahme der Papierprobe P, deren untere, der Probenaufnahmefläche 4 zugewandte Oberfläche vermessen werden soll. Die Probenaufnahmefläche 4 besteht in an sich bekannten Weise aus einem sehr glatten und ebenen Material, beispielsweise Glas und weist in ihrer Innenseite eine Bohrung 11 auf, über die sie mit dem Innenraum eines Behälters 2 verbunden ist. Das Volumen dieses Behälters ist dadurch veränderbar, daß er einen Messkolben 3 enthält, der in dem Behälter 2 verschiebbar ist. Die Verschiebung des Messkolbens 3 erfolgt über einen Schrittmotor 7 und einem Antrieb 12, im dargestellten Ausführungsbeispiel ein Riemenantrieb und über eine nicht im einzelnen dargestellte Spindel. Über die Verschiebung des Messkolbens 3 innerhalb des Behälters 2 lassen sich in dem Behälter 2 beliebige Volumina einstellen, konstant halten oder verändern. Der Behälter 2 weist darüber hinaus einen Drucksensor 10 zur Erfassung des aktuellen Behälterinnendrucks auf.

Die gesamte Anordnung aus Behälter 2 mit Messkolben 3 und Drucksensor 10 sowie Schrittmotor 7 und Antrieb 12 ist über einen Exzenterantrieb, bestehend aus einem Motor 8 und einem Exzenter 9 nach oben und unten Verschwenkbar, um den Spalt zwischen Probenaufnahmefläche 4 und Gummiplatte schließen zu können. Das Andrücken der Probe P erfolgt also durch Verschwenkung des im Bild unten dargestellten Teils der Messvorrichtung nach oben gegen den Druckstempel 1 und der Gummiplatte 5.

Verschiedene, mit der beschriebenen Vorrichtung ausführbaren Messverfahren werden im folgenden beschrieben.

Ein mit dem Verfahren nach Bekk konformer Messvorgang beginnt dadurch, daß bei geöffnetem Spalt (Exzenterantrieb 8 und 9 in unter Position) der Messkolben 3 nach oben auf Anschlag gefahren wird und damit das Volumen des Behälters auf Null gestellt wird. Nach dieser Initialisierung wird mit dem Messkolben 3 über den Schrittmotor 7 ein bestimmtes, definiertes Volumen, z. B. 160 ml eingestellt. Anschließend wird über den Exzenterantrieb 8, 9 der Behälter 2 angehoben und damit die Probe zwischen Probenaufnahmefläche 4 und Gummiplatte 5 eingeklemmt. Nun wird mittels des Schrittmotors 7 das Volumen des Behälters 2 auf 380 ml vergrößert, wodurch ein Unterdruck von ca. 520 mbar an der Messstelle erzeugt wird. Dieser Unterdruck wird vom Druchsensor 10 erfaßt und über eine (nicht dargestellte) Auswerteschaltung kontrolliert und angezeigt. Der Unterdruck in dem Behälter nimmt nun durch die entlang der zu messenden Oberfläche der Probe P ab, sobald er einen Wert von 507 mbar erreicht beginnt die Zeitmessung. Es wird also eine (nicht dargestellte) digitale Stopuhr gestartet und die verstrichene Zeit an einer Anzeigevorrichtung angezeigt. Erreicht der Unterdruck anschließend 480 mbar stoppt die Zeitmessung und das Ergebnis wird in sogenannten "Bekk-Sekunden" als unmittelbarer Wert für die Glätte der geprüften Papieroberfläche angezeigt.

Der wesentliche Unterschied des hier beschriebenen Messverfahrens - verglichen mit dem bekannten Bekk-Verfahren - besteht darin, daß das Volumen der Messkammer, also des Behälters 2 vergrößert werden kann. Hierdurch erspart man sich eine eigene Unterdruckpumpe. Vielmehr wird der Unterdruck durch Regelung des Volumens des Behälters 2 eingestellt und verändert.

Mit der beschriebenen Messvorrichtung lassen sich jedoch noch weitere, verbesserte Verfahren ausführen, die insbesondere wesentlich schneller Ergebnisse liefern, beispielsweise das Folgende.

Die Initialisierung der Vorrichtung erfolgt in der oben beschriebenen Weise durch hochfahren des Messkolbens in die obere Position, um das Volumen des Behälters 2 auf Null zu bringen und anschließend die Einstellung eines bestimmten Ausgangsvolumens, welches jedoch wesentlich geringer ist als das normale Volumen, beispielsweise nur 14 ml. Anschließend wir die Probe eingebracht und der Exzenter-Motor-Antrieb 8, 9 betätigt, um die Probe festzuklemmen.

Anschließend wird das Volumen des Behälters 2 so lange vergrößert, bis sich ein Unterdruck von 520 mbar einstellt, welcher über den Drucksensor 10 erfaßt und angezeigt wird. Dieser Volumenwert wird zwischengespeichert. Erreicht der Unterdruck durch Einströmen von Luft entlang der zu messenden Oberfläche einen Wert von 507 mbar startet - wie oben beschrieben - die Zeitmessung. Erreicht der Unterdruck anschließend einen Wert von 480 mbar stoppt die Zeitmessung und das Messverfahren ist in wesentlich kürzerer Zeit beendet, da nur ein geringes Ausgangsvolumen eingestellt wurde. Liegt jedoch der gemessene Wert für die Zeit unterhalb eines bestimmten Grenzwerts, so daß man nicht mehr von einer aussagekräftigen und genauen Messung ausgehen kann (dies passiert bei rauhen Papieren sehr leicht, da dann die Messzeit gering ist), so muß die Messung mit einem vergrößerten Volumen wiederholt werden. Dies erfolgt automatisch. Das Volumen des Behälters wird durch Absenken des Kolbens um einen bestimmten Wert erhöht (Inkrementwert), der neue Volumenwert abgespeichert und der Messvorgang wiederholt sich. Diese Wiederholung erfolgt sukzessiv so lange, bis die Zeit einen vorbestimmten Grenzwert überschritten hat. Aus dem Ergebnis (abgespeicherter Volumenwert und Messzeit) läßt sich der Bekk-Wert errechnen. Dieser wird angezeigt.

Die Vorteile des neuen Verfahrens liegen in sehr kurzen Messzeiten und in der automatischen Wiederholfunktion. Weitere Vorteile bestehen darin, daß keinerlei Ventile und Verschlauchungen mehr vorgesehen sind, und somit eine hohen Dichtigkeit und Zuverlässigkeit des Systems gegeben ist. Außerdem entfällt eine zweite Volumenkammer für den kleineren Messbereich, dadurch den Behälter 2 beliebige Volumina eingestellt werden können.

In einer weiteren Variante des Messverfahrens kann der Unterdruck konstant gehalten werden, und zwar durch nachregeln des Volumens des Behälters. Hierbei wird der Unterdruck in dem Behälter 2 von dem Drucksensor 10 kontinuierlich erfaßt und der Schrittmotor 7 wird von der Regelung angesteuert, so daß durch das Verschieben des Messkolbens 3 in dem Behälter 2 der Unterdruck in dem Behälter 2 auf einem konstanten Wert, z. B. 500 mbar gehalten wird. Bei diesem konstantem Unterdruck muß nur die Zeit gemessen werden, bis ein bestimmtes Volumen von z. B. 10 ml eingesaugt ist. Dieser Wert kann dann tabellarisch in Bekk-Sekunden umgerechnet werden, nachdem die Vorrichtung entsprechend geeicht wurde.

## Patentansprüche

1. Vorrichtung zur Bestimmung der Glätte der Oberfläche einer Papierprobe (P), mit einer ebenen, die Probe (P) tragenden Probenaufnahmefläche (4), innerhalb welcher sich eine zu einem evakuierbaren Behälter (2) führende Bohrung (11) befindet und einem auf die gegenüberliegende Oberfläche der Papierprobe (P) wirkenden, abdichtend ausgebildeten Druckstempel (1) zur Erzeugung eines im wesentlichen konstanten Anpressdrucks der Probe (P) auf die Probenaufnahmefläche (4), **dadurch gekennzeichnet, dass** das Volumen des Behälters (2) veränderbar ist.

2. Vorrichtung nach Anspruch 1, **dadurch gekennzeichnet, dass** der Behälter (2) einen darin befindlichen Messkolben (3) aufweist, welcher zur Veränderung des Volumens innerhalb des Behälters (2) verfahrbar ist.

3. Vorrichtung nach Anspruch 2, **dadurch gekennzeichnet, dass** das sie einen Schrittmotor (7) und einen Antrieb (12) zum Verfahren des Messkolbens (3) aufweist.

4. Vorrichtung nach einem der voranstehenden Ansprüche, **dadurch gekennzeichnet, dass** die Anordnung aus Probenaufnahmefläche (4), Behälter (2), Messkolben (3), Schrittmotor (7) und Antrieb (12) über einen Exzenterantrieb (8, 9) höhenverstellbar und gegen den gehäusefesten Druckstempel (1) andrückbar ist.

5. Verfahren zur Bestimmung der Glätte der Oberfläche einer Papierprobe (P), die auf eine ebene Probenaufnahmefläche (4) aufgelegt und abdichtend darauf angepresst wird, wobei durch eine zu einem evakuierbaren Behälter (2) führende Bohrung (11) innerhalb der Probenaufnahmefläche (4) ein Unterdruck angelegt wird, **dadurch gekennzeichnet, dass** zunächst das Ausgangsvolumen des Behälters (2) auf einen vorbestimmten Wert eingestellt wird, nach dem Einbringen und Anpressen der Probe (P) das Volumen des Behälters (2) bis zum Erreichen eines bestimmten Unterdrucks in dem Behälter (2) vergrößert wird, und während der Verringerung des Unterdrucks durch Einströmen von Luft entlang der Oberfläche der Probe (P) die Zeit zwischen zwei bestimmten Druckwerten als Wert für die Glätte gemessen wird.

6. Verfahren zur Bestimmung der Glätte der Oberfläche einer Papierprobe (P), die auf eine ebene Probenaufnahmefläche (4) aufgelegt und abdichtend darauf angepresst wird, wobei durch eine zu einem evakuierbaren Behälter (2) führende Bohrung (4) innerhalb der Probenaufnahmefläche (4) so lange ein Unterdruck angelegt wird, bis ein bestimmtes Luftvolumen zwischen Probenaufnahmefläche (4) und Oberfläche angesaugt wurde und diese Zeit als Wert für die zu messende Glätte dient, **dadurch gekennzeichnet, dass** zunächst das Ausgangsvolumen des Behälters (2) auf einen vorbestimmten Wert eingestellt wird, nach dem Einbringen und Anpressen der Probe (P) das Volumen des Behälters (2) bis zum Erreichen eines bestimmten Unterdrucks in dem Behälter (2) vergrößert und dieser Unterdruck regelungstechnisch konstant gehalten wird, und die Zeit für das Einströmen eines definierten Luftvolumens bei diesem konstanten Unterdruck als Wert für die Glätte gemessen wird.

7. Verfahren nach einem der Ansprüche 5 oder 6, **dadurch gekennzeichnet, dass** bei einem gemessenen Wert unterhalb eines vorbestimmten Grenzwertes das Ausgangsvolumen des Behälters (2) um ein vorbestimmtes Inkrement erhöht und anschließend der Messvorgang so lange wiederholt wird, bis der gemessene Wert oberhalb des vorbestimmten Grenzwertes liegt.
